# EUROPÄISCHE PATENTSCHRIFT

(11) **EP 0 584 126 B1**
(45) Veröffentlichungstag und Bekanntmachung des Hinweises auf die Patenterteilung: **16.04.1997**
(21) Anmeldenummer: 92909529.7
(22) Anmeldetag: 02.05.1992
(51) Int. Cl.: A61K 47/44, A61K 47/14, A61K 47/10

(54) **LANOLINDERIVATE ALS PENETRATIONSFÖRDERNDE SUBSTANZEN**
PENETRATION-PROMOTING SUBSTANCE
SUBSTANCES FACILITANT LA PENETRATION

(30) Priorität: 15.05.1991 DE 4115849
(43) Veröffentlichungstag der Anmeldung: 02.03.1994
(73) Patentinhaber: LTS LOHMANN Therapie-Systeme GmbH, 56567 Neuwied (DE)
(72) Erfinder: VON KLEINSORGEN, Reinhard, D-5450 Neuwied 22 (DE)
(74) Vertreter: Flaccus, Rolf-Dieter, Dr.
(86) Internationale Anmeldenummer: EP9200957
(87) Internationale Veröffentlichungsnummer: WO9220378

(56) Entgegenhaltungen:
- EP-A- 0 189 861
- EP-A- 0 370 481
- WO-A-87/00042
- AU-A- 520 987
- FR-A- 2 132 130
- GB-A- 2 176 105
- US-A- 5 057 497
- WORLD PATENTS INDEX LATEST Section Ch, Week 8611, Derwent Publications Ltd., London, GB; Class A, AN 86-073522; & JP-A-61 024 517
- FETTE-SEIFEN-ANSTRICHMITTEL, Bd. 85, Nr. 8, 1983, STUTTGART, Seiten 321-322; EL-NIMR A.E. ET AL: 'EFFECT OF WOOL WAXES AND WOOL WAX DERIVATIVES ON THE RELEASE OF SOME ANTIBIOTICS FROM A HYDROCARBON BASE'

## Beschreibung

Lanolinderivate als penetrationsfördernde Substanzen und ihre Verwendung in Arzneisubstanzen oder andere biologisch aktive Stoffe enthaltenden Formulierungen.

Die Erfindung betrifft Lanolinderivate in Mischung mit Polyethylenglykolethern längerkettiger Fettalkohole als penetrationsfördernde Substanzen in Arzneisubstanzen oder andere biologisch aktive Stoffe enthaltenden Formulierungen.

Die transdermale Applikation bietet für eine Vielzahl von pharmazeutischen Wirkstoffen oder anderen biologisch aktiven Stoffen eine Reihe von Vorteilen:
die Haut ist unbegrenzt zugänglich
es erfolgt kein Milieuwechsel wie bei peroraler Applikation
die Handhabung ist einfach und bequem
es genügt eine einmalige statt mehrfacher täglicher Gaben
es sind positive psychologische Effekte zu verzeichnen
es ist eine kontinuierliche Langzeitherapie möglich die Therapie kann jederzeit unterbrochen werden
es wird ein konstanter Plasmaspiegel über längere Zeit sichergestellt
ein Anfangs zu hoher Plasmaspiegel, wie bei intravenöser Applikation wird vermieden, wodurch eine geringe Nebenwirkungsrate auftritt
die Gefahr einer Über- oder Unterdosierung ist geringer
es wird eine kontrollierte Abgabe von Wirkstoffen insbesondere mit niedrigem therapeutischen Index sichergestellt

In vielen Fällen besitzen Arzneistoffe, die aufgrund ihres hohen "first-pass"-Effektes, ihrer niedrigen Dosierung und ihrer hohen Wirkpotenz ideale Kandidaten sind, eine derart geringe Hautpermeation, daß mit herkömmlichen Systemen eine Erzielung von therapeutischen Plasmawerten nicht möglich ist. Für all diese Arzneistoffe ist es notwendig, dem System sogenannte Penetrationsförderer zuzusetzen. In diesem Sinne sind eine Vielfalt von Substanzen beschrieben, aufgeführt in folgenden Patentschriften:
US 4 299 826, US 4 343 798, US 4 046 886, US 4 130 643, US 4 405 616, US 4 335 115, US 4 130 667, US 3 903 256, US 4 379 454, US 3 527 864, US 3 952 099, US 3 896 238, US 3 472 931.

Penetrationsfördernde Substanzen müssen neben ihrer spezifischen Aufgabe folgende Eigenschaften besitzen: Sie müssen auch bei längerem Verbleib auf der Haut unter okklusiven Bedingungen hautverträglich sein, dürfen kein allergenisierendes Potential besitzen und müssen Wirkstoffkompartibel sein.

Diese aus der Literatur bekannten Enhancer lassen sich verschiedenen chemischen Klassen zuordnen:
1. Primäre und sekundäre Alkohole
   1.1. Kurzkettige primäre Alkohole C₂ bis C₈
   1.2. Langkettige primäre Alkohole C₄ bis C₁₆
   1.3. Sekundäre Alkohole C₃ bis C₅
2. Anionische Tenside wie z.B. Na-Dodecylsulfat
3. Gesättigte und ungesättigte Fettsäuren
4. Azone und Derivate (1-Alkylazacycloheptan-2-on, 1-alkylazacycloalkanon
5. Amide wie N,N-Diethyl-3-methylbenzamid (DEET), N,N-diethyl-m-toluamide
6. Alkyl-N,N-dialkylaminoacetat
7. Macrocyclische Ketone und Lactone
8. Pyrrolidone
9. Ester wie Ethylacetat, Isopropylmyristat, Glycerinmonolaurat, Diethylsebacat.
   Propylenglykolester gesättigter Fettsäuren
10. Terpene wie Limonen, Menthol, Cineol
11. Phospholipide
12. Organische Säuren wie Citronensäure, Salicylsäure etc.
13. Kationische Tenside bzw. Amine

Die Vielzahl unterschiedlicher Stoffe verschiedenster chemischer Struktur mit nachgesagter penetrationsfördernder Wirkung läßt einen einzigen Wirkungsmechanismus als unwahrscheinlich erscheinen. So werden dann auch verschiedene Mechanismen bzw. Kombinationen von Mechanismen diskutiert.
1. Lösemitteleffekte bezogen auf Wirkstoff und Hautlipide.
2. Effekte auf die Lipidstruktur der Membran.
3. Effekte auf das Keratin und die Proteinstruktur der Haut.

Bei der Vielzahl von Wechselwirkungen innerhalb der Haut und der unterschiedlichen chemischen Natur der Wirkstoffe lassen sich die penetrationsfördernden Eigenschaften all dieser sogenannten Enhancer bezüglich eines Wirkstoffes nur schwer voraussagen.
Erfahrungsgemäß gilt, daß äußerst selten eine penetrationsfördernde Substanz bzw. ein bestimmtes Gemisch für mehrere Arzneistoffe oder Arzneistoffgruppen die geforderten Eigenschaften erfüllt.

Aus der JP-A-61024517 ist ein transdermales Therapie-System gegen Krankheiten der Kreislauforgane bekannt, umfassend ein Pflaster mit einer Klebschicht, eine penetrationsfördernde Substanz sowie einen Beta-Blocker als Wirkstoff. Als Penetrationsförderer wird Isopropylmyristat und/oder Isopropyllanolat verwendet. Als Vorteil wird eine langanhaltende Verabreichung der Beta-Blocker-Wirkstoffe angegeben, ohne Irritation der Haut. Der Wirkstoff erreicht den Blutkreislauf direkt ohne die Leber zu passieren, weshalb keine schädlichen Nebenwirkungen auftreten. Zur Herstellung wird die penetrationsfördernde Substanz als Dispersion in die Klebschicht eingearbeitet, die als Arzneistoff den Beta-Blocker enthält.

Aus der FR-A-21 32 130 sind kosmetische Zubereitungen wie Sonnencremes, Gesichts-, Körper- oder Handcremes insbesondere als Feuchtigkeitsspender bekannt. Es handelt sich um Emulsionen von "Wasser-in-Öl-Typ". Zur Stabilisierung dieser Emulsionen werden Mischungen von Lanolaten wie Magnesiumlanolat, Kalzium, Lithium, Zink und Aluminium-Lanolat verwendet. Ziel der Formulierung ist eine verbesserte Hydratation der Epidermis und ein besserer Schutz für diese. Nach Beispiel 1 enthält eine Formulierung beispielsweise Magnesiumlanolat, Alkohol von Lanolin, Isopropylpalmitat, Paraffin-Öl, Mandel-Öl, Ozokerit, Wasser und Parahydroxybenzoat von Methyl.
Die Verwendung von Polyethylenglycolethern längerkettiger Fettalkohole als Penetrationsverbesserer in transdermalen Systemen ist beispielsweise in der EP-A-0 189 861, Seite 10, Zeilen 16 bis 24, erwähnt. Es handelt sich bei diesen Penetrationsförderern beispielsweise um Polyoxyethylenalkylether, ausgewählt aus Alkylgruppen mit 4 bis 20, vozugsweise10 bis 18 Kohlenstoffatomen mit Zusatz von Ethylenoxid wie beispielsweise Polyoxyethylen-Laurylether, Polyoxyethylen-Cetylether, Polyoxyether-Stearylether und Polyoxyethylen-Oleylether. Eine Verwendung zusammen mit Lanolinderivaten ist jedochaus diesem Dokument weder bekannt noch nahegelegt.
Aus der WO/A/8700042 sind transdermale Systeme mit Isopropylmyristat als Penetra tionsverbesserer für Verapamil bekannt. Entsprechend in dem darin erwähnten Beispiel 10 wurde durch Vorbehandlung der Haut mit Isoproplymyristat vor dem Anlegen einer wirkstoffhaltigen Matrix eine vergleichsweise gute Penetration des Wirkstoffes durch die Haut beobachtet. Ein Vergleich gemäß Beispiel 11 mit in die Matrix eingearbeiteten Isopropylmyristat ergab eine erheblich geringere penetrationsfördernde Wirkung. für den Wirkstoff Verapamil ergab sich nach Beispiel 12 eine erheblich gesteigerte Permeationsrate.

Aufgabe der Erfindung ist die Bereitstellung von penetrationsfördernden Sustanzen, die hautverträglich und wirkstoffkompartibel sind sowie kein Allergenisierungspotential besitzen, darüber hinaus leicht zugänglich und wirtschaftlich sind und gleichzeitig penetrationsfördernde Wirkung auf mehr als einen Wirkstoff besitzen.

Überraschenderweise wurde nun gefunden, daß bestimmte Lanolinderivate die Eigenschaft aufweisen, die Penetration bestimmter Arzneistoffe bzw. Wirkstoffe durch die Haut zu erhöhen. Diese Stoffe werden üblicherweise in der Kosmetik zur Herstellung von Cremes und Lotion eingesetzt.

Diese Aufgabe wird erfindungsgemäß durch Verwendung von Lanolinderivaten zusammen mit Polyethylenglykolethern langkettiger Fettalkohole als penetrationsfördernde Substanzen in Arzneisubstanzen oder andere biologisch aktive Stoffe enthaltenden Formulierungen gelöst.

Bevorzugte Lanolinderivate sind ausgewählt aus der Gruppe, die aus acetyliertem Lanolin, acetyliertem Lanolinalkohol, alkoxyliertem Lanolin, Lanolinsäure, polyethoxylierter Lanolinsäure, polyethoxyliertem Lanolinalkohol, Ester der Lanolinsäure mit kurzkettigen aliphatischen Alkoholen wie Isopropyllanolat, Polyethylenglykolether des Lanolinalkohols und Ester des Lanolinalkohols mit langkettigen Fettsäuren besteht. Sofern polyethoxylierte Lanolinderivate verwendet werden, kann die Zahl der Ethylenoxydmolekule bei 2 bis 50 betragen.
Als Ester der Lanolinsäure mit kurzkettigen aliphatischen Alkoholen kommen in erster Linie die geraden oder verzweigten Alkohole mit C1 bis C4 in Frage, die bevorzugt primär oder sekundär sind. Beispiele hierfür sind Methanol, Ethanol, n-Propanol, n-Butanol, Isopropanol. Für Ester des Lanolinalkohols mit langkettigen Fettsäuren kommen in erster Linie die gesättigten oder ungesättigten eigentlichen Fettsäuren wie Laurinsäure, Palmitinsäure, Stearinsäure ebenso in Frage wie Myristinsäure, Palmitinsäure, Ölsäure und Linolsäure.

Werden in Verbindung mit Lanolinderivaten auch noch Ester des Isopropanols mit langkettigen Fettsäuren und/oder Polyethylenglykolethern längerkettiger Fettalkohole eingesetzt, so kommen als Fettsäurekomponenten der entsprechenden Isopropanolester die vorstehend genannten Fettsäuren in Frage. Als längerkettige typische Fettalkohole kommen die den oben genannten Fettsäuren entsprechenden Alkohole wie Oleylalkohol, Laurylalkohol, Cetylalkohol und Stearylalkohol, oder deren Polyethylenglykolethern in Frage, die aus den entsprechenden Alkoholen durch Umsetzung mit unterschiedlichen Molmengen Ethylenoxid erhalten werden. Bekannte Produkte sind die Kondensationsprodukte von Oleylalkohol mit 2 bis 50 Molen Ethylenoxid, von Laurylalkohol mit 2 bis 40, Cetylalkohol mit 2 bis 45 und Stearylalkohol mit 2 bis 100 Molen Ethylenoxid.

Bevorzugt besteht die penetrationsfördernde Substanz zu wenigstens 1 Gew.-%, insbesondere zu 1 bis 60 Gew.-% aus einem Lanolinderivat, und bis 99, insbesondere 30 bis 90 Gew.-% aus einem Polyethylenglykolether eines Fettalkohols (Summe der Komponenten gleich 100).

Eine Formulierung zur Verabreichung von Verapamil oder Gallopamil durch die Haut ist dadurch gekennzeichnet, daß zur Erhöhung der transdermalen Permeation als penetrationsfördernder Anteil Lanolinderivate allein oder in Mischung mit Polyethylenglykolethern längerkettiger Fettalkohole enthalten sind.

Sofern die penetrationsfördernde Substanz in einem therapeutischen transdermalen System (TTS) zur Anwendung kommt, so besteht dieses aus einer wirkstoffundurchlässigen Rückschicht, wenigstens einem daran anschließenden wirkstoffhaltigen Reservoir, in dem der penetrationsfördernde Anteil enthalten ist, einer Einrichtung zur Fixierung auf der Haut und gegebenenfalls einer wiederablösbaren Schutzschicht. Im einfachsten Fall liegt eine sogenannte Einschichtformulierung vor, bei der die penetrationsfördernde Substanz (zusammen mit dem Wirkstoff) in einer vorzugsweise selbstklebenden Matrix verteilt ist, die hautseitig mit einer abhäsiv ausgerichteten Schutzschicht und auf der der Haut abgewandten Seite mit einer Deckfolie versehen ist.

Neben einer derartigen Einschichtformulierung, in der der Penetrationsförderer aus einer Lösung oder Suspension in die vorzugsweise selbstklebende Matrix eingearbeitet wird, kann der Arzneistoff auch mit den penetrationsfördernden Substanzen verrieben werden, worauf die Mischung auf einen Träger, vorzugsweise ein Stück Vlies oder ein Gewebe oder ein Schaumstoff aufgebracht wird. Der Träger wird dann mittels einer Selbstklebefolie auf der Haut fixiert.

Darüber hinaus ist es aber auch möglich, ein mehrschichtiges TTS zu verwenden. Zum Beispiel kann in einem solchen Fall der Arzneistoff entweder allein oder mit einem Teil der penetrationsfördernden Substanz auf einem Träger angeordnet sein, der auf oder in einer, hautseitig gesehen, ersten Kleberschicht angeordnet ist, während entweder die Gesamtmenge des Penetrationsförderers oder jedenfalls ein Teil davon in einer vom Reservoir getrennten Schicht, vorzugsweise in der Klebeschicht der Deckfolie, verteilt ist. Es können somit die penetrationsfördernden Substanzen in verschiedenen Schichten in verschiedener Konzentration bzw. Menge vorliegen.

Es hat sich gezeigt, daß die erfindungsgemäß als Penetrationsförderer verwendeten Substanzen sowohl zusammen mit dem Arzneistoff in den dem Fachmann bekannten üblichen Matrixformulierungen mit selbstklebenden Eigenschaften, als auch gemeinsam mit dem Arznei- bzw. Wirkstoff in einem im therapeutischen System fixierten Gel, einer Creme oder auch Salbe eingesetzt und diese direkt mit der intakten Haut in Kontakt gebracht werden können.

Trotz mehrfach wiederholter Anwendung konnte keine Hautirritation festgestellt werden. Besonders vorteilhaft ist die penetrationsfördernde Wirkung mit den Wirkstoffen Verapamil und Gallopamil. Für Verapamil ist eine Penetrationsförderung von Isopropylmyristat aus der PCT/WO87/00042 vorbeschrieben. Die erfindungsgemäß verwendeten Lanolinderivate weisen jedoch eine wesentlich stärkere penetrationsfördernde Wirkung für Verapamil auf, wie die folgenden Beispiele zeigen.

Die Erfindung wird durch die nachfolgenden Beispiele näher erläutert:

### A. Einschicht-Formulierung

Bei den Rezepturen unter der Bezeichnung "Einschicht-Formulierung" handelt es sich um selbstklebende Matrix-Formulierungen mit folgendem Aufbau des TTS (siehe Figur):
Auf einer abhäsiv ausgerüsteten Schutzschicht (1) ist die selbstklebende Matrix (2) angeordnet, die durch eine Deckfolie (3) abgedeckt ist.

### Einschicht-Formulierung Beispiel 1:

Ein pharmazeutisches Produkt gemäß der vorliegenden Erfindung mit einem einschichtigen Aufbau der wirkstoffhaltigen Klebermatrix wird wie folgt hergestellt:
Eine die penetrationsfördernde Komponente und den Arzneistoff beinhaltende Haftklebermasse, bestehend aus:
- 0,170 kg: Polyisobutylen (mittleres Molekulargewicht von 900.000 bis 1.400.000)
- 0,202 kg: festes aliphatisches Kohlenwasserstoffharz (Handelsname Hercures C, MG ca. 1100)
- 0,152 kg: Polyterpenharz
- 0,072 kg: Polymer von Ethylenoxyd HO(Ch₂-Ch₂-O)ₙH n=200 (PEG 200)
- 0,072 kg: Kolloidales Siliciumdioxyd
- 0,079 kg: Isopropyllanolat
- 0,072 kg: Isopropylmyristat
- 0,181 kg: Gailopamil
- 1,200 kg: Spezialbenzin 80-110 als Lösungsmittel
wird so auf eine einseitig mit Aluminium bedampfte und beidseitig abhäsiv ausgerüstete Schutzschicht aufgetragen, daß nach Abdampfen des Lösungsmittels eine haftklebende Schicht von 82 g/m² erhalten wird.

Nach Abdecken der haftklebenden Schicht mit einer undurchlässigen Deckschicht, bestehend aus einem Polyester, wird das erhaltene Laminat den therapeutischen Erfordernissen entsprechend in Einzelstücke aufgeteilt.

### Ergebnis Beispiel 1:

- Gehalt:: 14,80 mg/10 cm² Gallopamil
- Penetration (Mäusehaut):: 9,61 mg Gallopamil/10 cm²/24 h

### Einschicht-Formulierung Beispiel 2:

Herstellung entsprechend Beispiel 1:

### Zusammensetzung:

- 0,213 kg: Polyisobutylen
- 0,253 kg: Kohlenwasserstoffharz
- 0,190 kg: Polyterpenharz
- 0,045 kg: PEG 200
- 0,091 kg: Aerosil 200
- 0,050 kg: Isopropylanolat
- 0,045 kg: Isopropylmyristat
- 0,113 kg: Verapamil
- 1,400 kg: Spezialbenzin 80-110

Haftklebende Schicht nach
Abdampfen des Lösungsmittels: 74 g/m²

### Ergebnis Beispiel 2:

- Gehalt:: 8,4 mg Verapamil/10 cm²
- Penetration (Mäusehaut):: 5,71 mg Verapamil/10 cm²/24 h

### Einschicht-Formulierung Beispiel 3:

Herstellung entsprechend Beispiel 1.

### Zusammensetzung:

- 0,213 kg: Polyisobutylen
- 0,253 kg: Kohlenwasserstoffharz
- 0,190 kg: Polyterpenharz
- 0,045 kg: PEG 200
- 0,091 kg: Aerosil 200
- 0,050 kg: Isopropyllanolat
- 0,045 kg: POÄ (10) Oleylalkoholether
- 0,113 kg: Verapamil
- 1,300 kg: Spezialbenzin 80-110

Haftklebende Schicht nach
Abdampfen des Lösungsmittels: 85 g/m²

### Ergebnis Beispiel 3:

- Gehalt:: 9,62 mg Verapamil/10 cm²
- Penetration (Mäusehaut):: 6,14 mg Verapamil/10 cm²/24 h

### Einschicht-Formulierung Beispiel 4 (Referenzbeispiel):

Herstellung entsprechend Beispiel 1.

### Zusammensetzung:

- 0,223 kg: Polyisobutylen
- 0,265 kg: Kohlenwasserstoffharz
- 0,199 kg: Polyterpenharz
- 0,047 kg: PEG 200
- 0,095 kg: Aerosil 200
- 0,050 kg: Isopropyllanolat
- 0,119 kg: Gallopamil
- 1,210 kg: Spezialbenzin 80-110

Haftklebende Schicht nach
Abdampfen des Lösungsmittels: 75 g/m²

### Ergebnis Beispiel 4:

- Gehalt: 8,89 mg Gallopamil/10 cm²
- Penetration (Mäusehaut):: 5,71 mg Gallopamil/10 cm²/24 h

Zur Herstellung weiterer selbstklebender Matrix-Formulierungen werden die in der Tabelle angegebenen Substanzen in Form ihrer Lösung bzw. Suspension (Lösungs- bzw. Dispersionsmittel; Benzin) gemischt, auf die abhäsiv ausgerüstete Schutzschicht mittels einer Beschichtungseinrichtung aufgetragen, durch Erhitzen von Lösungsmittel befreit und mit der Deckfolie kaschiert. Das Trockengewicht der selbstklebenden Matrix (FG) ist in der tabellarischen Übersicht in g/m² angegeben. (T bedeutet Gewichtsteile).
Schutzschicht und Deckschicht sind die gleichen wie in Beispielen 1 bis 4.

Die in der genannten Beispiel sind als Referenzversuche zur alleinigen Verwendung eines Penetrationsbeschleunigers in der Beschreibung belassen.

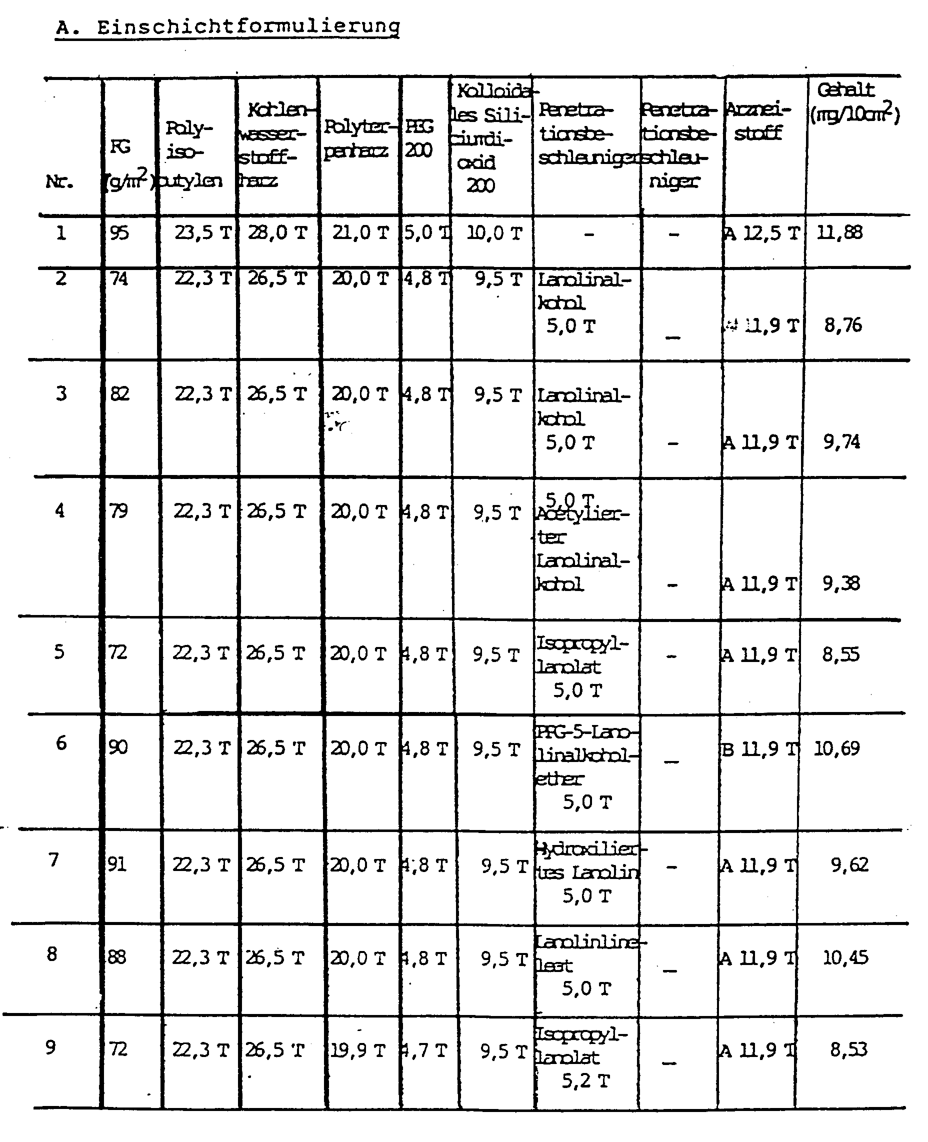

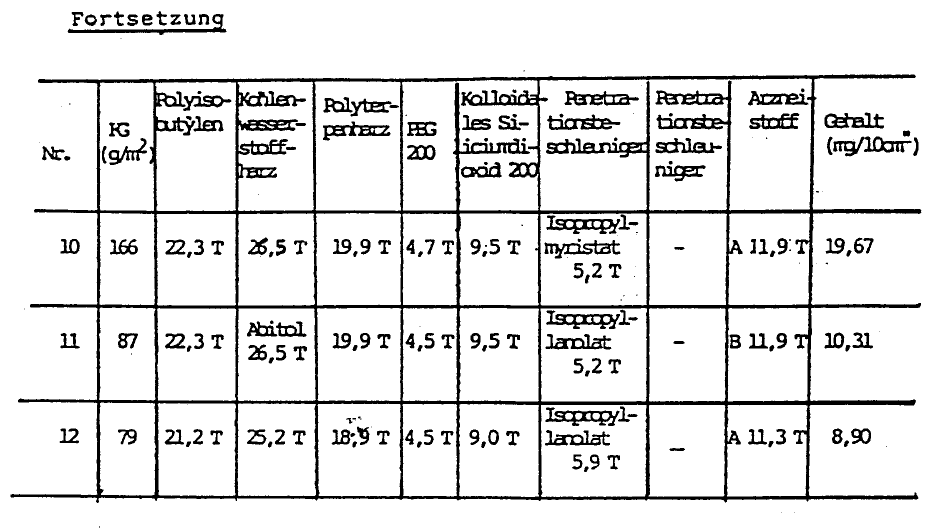

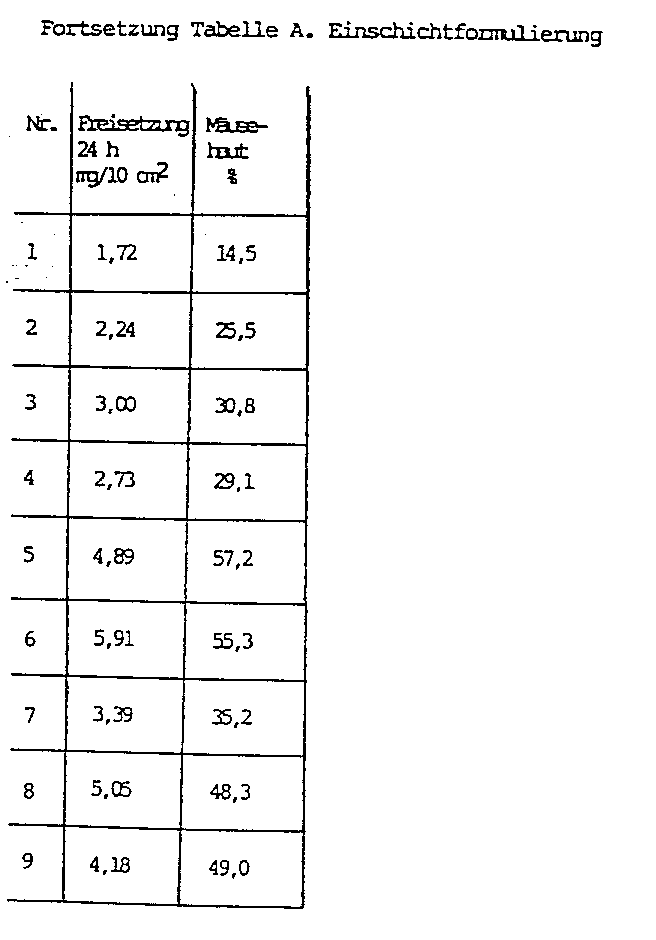

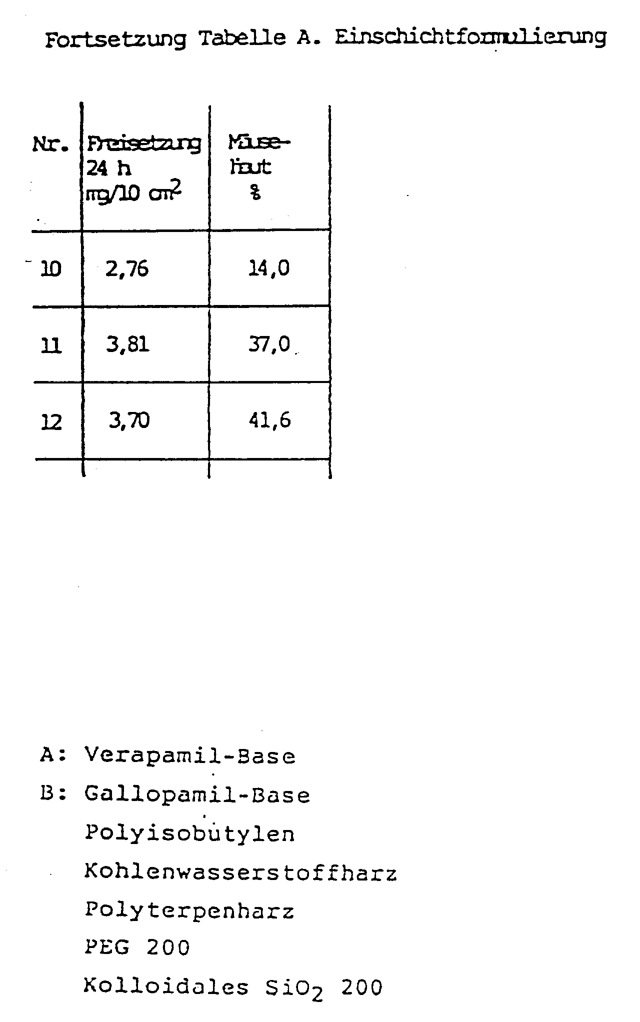

### B. Reservoir-Formulierung (Verreibungen)

Bei den Rezepturen unter der B.) handelt es sich um Verreibungen des Arzneistoffes mit den in der Tabelle angegebenen penetrationsfördernden Substanzen. Zur Herstellung eines TTS werden diese Mischungen in der in der Tabelle angegebenen Konzentration auf einen Träger aufgetragen.

Dieser Träger kann bestehen aus:
Gewebe
Vlies
Schaumstoff (offenporig).

Eine derartige mit der Verreibung getränkte Gewebe-, Vlies oder Schaumstoffscheibe wird mittels einer selbstklebenden Folie auf der Haut fixiert.

### Beispiel 13:

Auf ein Pflaster, bestehend aus einer selbsthaftenden Deckschicht und einem zentralen Vlies, werden auf das Vlies 130 mg einer Mischung (Verreibung), bestehend aus:
- 2,0 g: Verapamil
- 1,0 g: Isopropyllanolat
- 1,0 g: POÄ (10) Oleylalkoholether
- 1,0 g: Isopropylmyristat
aufgetragen.

Penetration des Arzneistoffes durch die Mäusehaut nach Applikation: 15,26 mg Verapamil/2,54 cm²/24 h

### Beispiel 14 (Referenzbeispiel):

Auf ein Pflaster, bestehend aus einer selbsthaftenden Deckschicht und einem zentralen Vlies, werden auf das Vlies 53 mg einer Mischung (Verreibung), bestehend aus:
- 2,0 g: Verapamil
- 1,0 g: Isopropyllanolat
aufgetragen.

Penetration des Arzneistoffes durch die Mäusehaut nach Applikation: 6,76 mg Verapamil/2,54 cm²/24 h

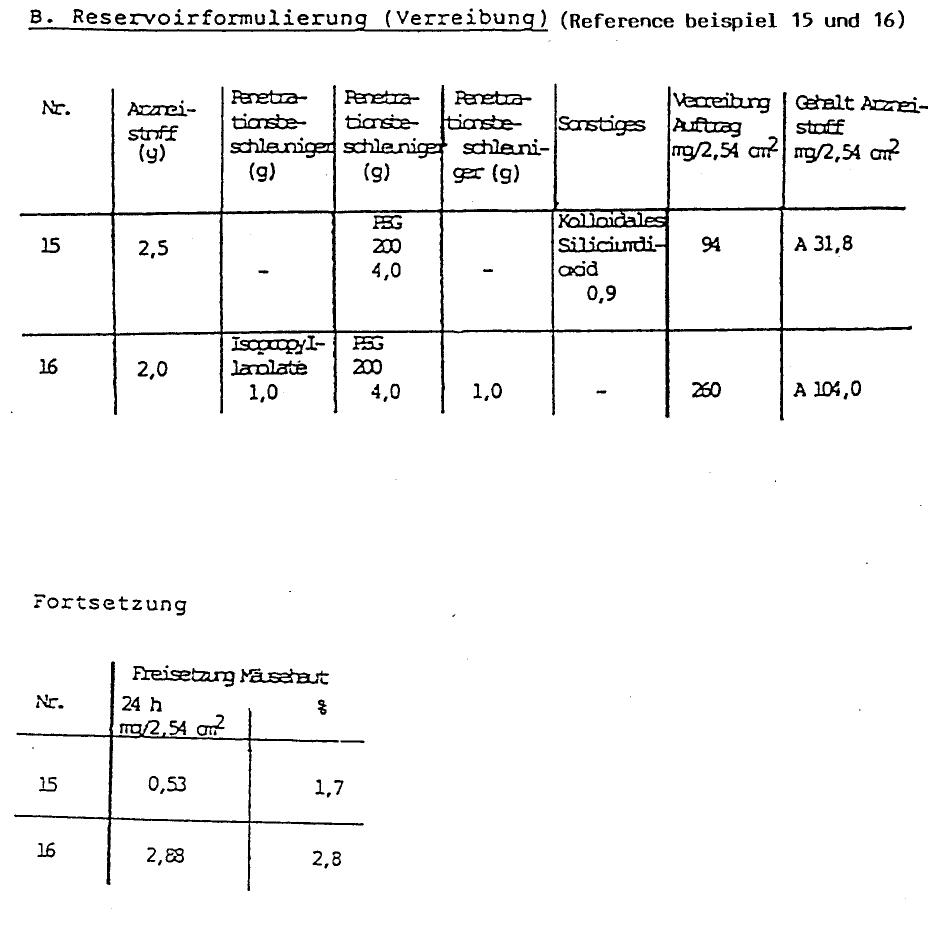

### C. Mehrschichtformulierung

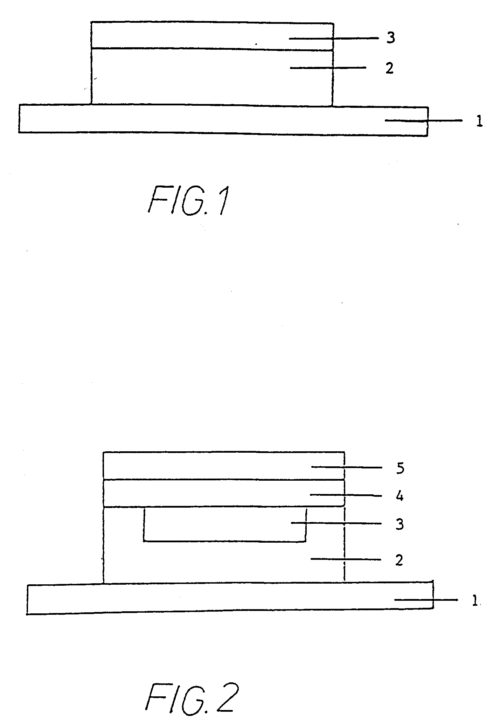

Die nachfolgend beschriebenen Mehrschichtformulierungen weisen folgenden Aufbau auf (siehe Fig. 2):

Auf einer abhäsiv ausgerüsteten Schutzfolie (1) ist eine Kleberschicht (2) angeordnet, auf bzw. in der das Reservoir (3) angeordnet ist. Das Reservoir (3) besteht aus einer Klebscheibe, die mit dem Arzneistoff und einem Polyethylenglykolether des Oleylalkohols imprägniert ist. Das Reservoir (3) ist mit einer mit Kleber (4) beschichteten Deckfolie (5) abgedeckt.

Zur Herstellung eines solchen Systems wird wie folgt verfahren:

Die mit dem Kleber (2) beschichtete abhäsiv ausgerüstete Schutzfolie (1) wird mit einer Vliesstoff-Scheibe (3) versehen. Diese Vliesstoff-Scheibe wird mit der Wirkstoff-Formulierung dotiert. Anschließend wird die mit Kleber beschichtete Deckfolie (5) aufkaschiert.

### Beispiel 17:

### Herstellung:

Eine einseitig aluminisierte und beidseitig abhäsiv ausgerüstete Schutzfolie wird derart mit einer Mischung, bestehend aus:
- 71,1 g: Polyacrylat-Kleber-Lsg.
- 32,2 g: Polyacrylat basisch
- 6,7 g: Isopropyllanolat
beschichtet, daß nach Abdampfen des Lösungsmittels ein Flächengewicht der adhäsiven Schicht von 50 g/m² resultiert (Kleber 2-Matrix).

Auf diese adhäsive Schicht wird eine Vliesscheibe aufgelegt, die mit einer Mischung, bestehend aus gleichen Anteilen Verapamil und POÄ (10) Oleylalkoholether, dotiert wird.

Konzentration an Verapamil im Vlies nach Dotierung:
65,3 mg/13,85 cm².

Das dotierte Vlies und die beschichtete Schutzfolie werden mit einer selbsthaftenden Deckfolie, bestehend aus einer Polyacrylat-Matrix mit einem Flächengewicht von 100g/m2 und einer Polyester-Folie derart abgedeckt (laminiert), daß das dotierte Vlies von den beiden selbsthaftenden Matrices eingeschlossen ist. Das erhaltene Laminat wird derart gestanzt, daß neben dem Vlies ein 1 cm breiter wirkstofffreier adhäsiver Rand verbleibt.
- Mäusehautpenetration:: 3,78 mg Verapamil/2,54 cm²/24 h

## Patentansprüche (Patentansprüche für folgende(n) Vertragsstaat(en): AT, BE, CH, DE, DK, FR, GB, IT, LI, LU, NL, SE)

1. Verwendung von Lanolinderivaten zusammen mit Polyethylenglykolethern längerkettiger Fettalkohole als penetrationsfördernde Substanzen in Arzneisubstanzen oder andere biologisch aktive Stoffe enthaltenden Formulierungen.

2. Formulierung zur Erhöhung der transdermalen Permeation von Arzneisubstanzen oder anderen biologisch aktiven Stoffen, gekennzeichnet durch einen Gehalt an einem penetrationsfördernden Anteil von Lanolinderivaten zusammen mit Polyethylenglykolether längerkettiger Fettalkohole als penetrationsfördernder Substanz.

3. Formulierung nach Anspruch 2, dadurch gekennzeichnet, daß die Lanolinderivate ausgewählt sind aus der Gruppe bestehend aus acetyliertem Lanolin, acetyliertem Lanolinalkohol, alkoxyliertem Lanolin, Lanolinsäure, polyethoxylierter Lanolinsäure, polyethoxyliertem Lanolinalkohol, Ester der Lanolinsäure mit kurzkettigen aliphatischen Alkoholen wie Isopropyllanolat und Ester des Lanolinalkohols mit langkettigen Fettsäuren.

4. Formulierung nach Anspruch 2, dadurch gekennzeichnet, daß die Polyethylenglykolether längerkettiger Fettalkohole ausgewählt sind aus der Gruppe, bestehend aus Umsetzungsprodukten von Oleylalkohol mit 2 bis 50 Molen Ethylenoxid, Laurylalkohol mit 2 bis 40 Molen Ethylenoxid, Cetylalkohol mit 2 bis 45 Molen Ethylenoxid und Stearylalkohol mit 2 bis 100 Molen Ethylenoxid.

5. Formulierung nach einem der Ansprüche 1 bis 4, dadurch gekennzeichnet, daß die penetrationsfördernde Substanz zu wenigstens 1 Gew.-%, vorzugsweise 1 bis 60 Gew.-% aus einem Lanolinderivat und bis 99 Gew.-%, vorzugsweise 30 bis 90 Gew.% aus einem Polyethylenglykolether längerkettiger Fettalkohole besteht, wobei die Summe der Komponenten immer 100 beträgt.

6. Formulierung zur Verabreichung von Verapamil oder Gallopamil durch die Haut, dadurch gekennzeichnet, daß zur Erhöhung der transdermalen Permeation als penetrationsfördernder Anteil Lanolinderivate in Mischung mit Polyethylenglykolethern längerkettiger Fettalkohole enthalten sind.

7. Verwendung von Lanolinderivaten, vorzugsweise ausgewählt aus der Gruppe, bestehend aus acetyliertem Lanolin, acetyliertem Lanolinalkohol, alkoxyliertem Lanolin, Lanolinsäure, polyethoxylierter Lanolinsäure, polyethoxyliertem Lanolinalkohol, Ester der Lanolinsäure mit kurzkettigen aliphatischen Alkoholen wie Isopropyllanolat und Ester des Lanolinalkohols mit langkettigen Fettsäuren in Mischung mit Estern des Isopropylalkohols mit langkettigen Fettsäuren, vorzugsweise ausgewählt aus der Gruppe bestehend aus Isopropylisostearat, Isopropyllaurat, Isopropyllinoleat, Isopropylmyristat, Isopropylpalmitat und Isopropylstearat und/oder mit Polyethylenglykolethern längerkettiger Fettalkohole, vorzugsweise ausgewählt aus der Gruppe, bestehend aus Umsetzungsprodukten von Oleylalkohol mit 2 bis 50 Molen Ethylenoxid, Laurylalkohol mit 2 bis 40 Molen Ethylenoxid, Cetylalkohol mit 2 bis 45 Molen Ethylenoxid und Stearylalkohol mit 2 bis 100 Molen Ethylenoxid als penetrationsfördernde Substanzen für Verapamil oder Gallopamil enthaltende Formulierungen.

8. Transdermales therapeutisches System zur Verabreichung von Gallopamil oder Verapamil, das eine Formulierung nach einem der Ansprüche 2 bis 6 enthält.

## Patentansprüche (Patentansprüche für folgende(n) Vertragsstaat(en): ES, GR)

1. Verfahren zur Verwendung von Lanolinderivaten zusammen mit Polyethylenglykolethern längerkettiger Fettalkohole als penetrationsfördernde Substanzen in Arzneisubstanzen oder andere biologisch aktive Stoffe enthaltenden Formulierungen.

2. Verfahren zur Herstellung einer Formulierung zur Erhöhung der transdermalen Permeation von Arzneisubstanzen oder anderen biologisch aktiven Stoffen, gekennzeichnet durch einen Gehalt an einem penetrationsfördernden Anteil von Lanolinderivaten zusammen mit Polyethylenglykolether längerkettiger Fettalkohole als penetrationsfördernder Substanz.

3. Verfahren zur Herstellung einer Formulierung nach Anspruch 2, dadurch gekennzeichnet, daß die Lanolinderivate ausgewählt sind aus der Gruppe bestehend aus acetyliertem Lanolin, acetyliertem Lanolinalkohol, alkoxyliertem Lanolin, Lanolinsäure, polyethoxylierter Lanolinsäure, polyethoxyliertem Lanolinalkohol, Ester der Lanolinsäure mit kurzkettigen aliphatischen Alkoholen wie Isopropyllanolat und Ester des Lanolinalkohols mit langkettigen Fettsäuren.

4. Verfahren zur Herstellung einer Formulierung nach Anspruch 2, dadurch gekennzeichnet, daß die Polyethylenglykolether längerkettiger Fettalkohole ausgewählt sind aus der Gruppe, bestehend aus Umsetzungsprodukten von Oleylalkohol mit 2 bis 50 Molen Ethylenoxid, Laurylalkohol mit 2 bis 40 Molen Ethylenoxid, Cetylalkohol mit 2 bis 45 Molen Ethylenoxid und Stearylalkohol mit 2 bis 100 Molen Ethylenoxid.

5. Verfahren zur Herstellung einer Formulierung nach einem der Ansprüche 1 bis 4, dadurch gekennzeichnet, daß die penetrationsfördernde Substanz zu wenigstens 1 Gew.-%, vorzugsweise 1 bis 60 Gew.-% aus einem Lanolinderivat und bis 99 Gew.-%, vorzugsweise 30 bis 90 Gew.% aus einem Polyethylenglykolether längerkettiger Fettalkohole besteht, wobei die Summe der Komponenten immer 100 beträgt.

6. Verfahren zur Herstellung einer Formulierung zur Verabreichung von Verapamil oder Gallopamil durch die Haut, dadurch gekennzeichnet, daß zur Erhöhung der transdermalen Permeation als penetrationsfördernder Anteil Lanolinderivate in Mischung mit Polyethylenglykolethern längerkettiger Fettalkohole enthalten sind.

7. Verfahren zur Verwendung von Lanolinderivaten, vorzugsweise ausgewählt aus der Gruppe, bestehend aus acetyliertem Lanolin, acetyliertem Lanolinalkohol, alkoxyliertem Lanolin, Lanolinsäure, polyethoxylierter Lanolinsäure, polyethoxyliertem Lanolinalkohol, Ester der Lanolinsäure mit kurzkettigen aliphatischen Alkoholen wie Isopropyllanolat und Ester des Lanolinalkohols mit langkettigen Fettsäuren in Mischung mit Estern des Isopropylalkohols mit langkettigen Fettsäuren, vorzugsweise ausgewählt aus der Gruppe bestehend aus Isopropylisostearat, Isopropyllaurat, Isopropyllinoleat, Isopropylmyristat, Isopropylpalmitat und Isopropylstearat und/oder mit Polyethylenglykolethern längerkettiger Fettalkohole, vorzugsweise ausgewählt aus der Gruppe, bestehend aus Umsetzungsprodukten von Oleylalkohol mit 2 bis 50 Molen Ethylenoxid, Laurylalkohol mit 2 bis 40 Molen Ethylenoxid, Cetylalkohol mit 2 bis 45 Molen Ethylenoxid und Stearylalkohol mit 2 bis 100 Molen Ethylenoxid als penetrationsfördernde Substanzen für Verapamil oder Gallopamil enthaltende Formulierungen.

8. Verfahren zur Herstellung eines Transdermalen therapeutischen Systems zur Verabreichung von Gallopamil oder Verapamil, das eine Formulierung nach einem der Ansprüche 2 bis 6 enthält.

## Claims (Claims for the following Contracting State(s): AT, BE, CH, DE, DK, FR, GB, IT, LI, LU, NL, SE)

1. Use of lanoline derivatives together with polyethyleneglycol ethers of fatty alcohols with longer chains as penetration enhancing substances in formulations containing pharmaceutical substances or other, biologically active substances.

2. Formulation to increase the transdermal permeation of pharmaceutical substances or other biologically active substances characterised by a content of a penetration enhancing portion of lanoline derivatives together with polyethyleneglycol ethers of fatty alcohols with longer chains as penetration enhancing substances.

3. Formulation according to Claim 2, characterised in that the lanoline derivatives are selected from the group consisting of acetylated lanoline, acetylated lanoline alcohol, alkoxylated lanoline, lanoline acid, polyethoxylated lanoline acid, polyethoxylated lanoline alcohol, esters of lanoline acid with short-chain aliphatic alcohols such as isopropyl lanolate and esters of lanoline alcohol with long-chain fatty acids.

4. Formulation according to Claim 2, characterised in that the polyethyleneglycol ethers of fatty alcohols with longer chains are selected from the group consisting of reaction products of oleyl alcohol with 2 - 50 moles of ethylene oxide, lauryl alcohol with 2 - 40 moles of ethylene oxide, cetyl alcohol with 2 - 45 moles of ethylene oxide and stearyl alcohol with 2 - 100 moles of ethylene oxide.

5. Formulation according to any one of Claims 1 to 4, characterized in that the penetration enhancing substance consists of at least 1%-wt., preferably 1 to 60%-wt., of a lanoline derivative, and of up to 99%-wt., preferably 30 to 90%-wt, of a polyethyleneglycol ether of fatty alcohols with longer chains, the sum of the components always amounting to 100.

6. Formulation for the administration of verapamile or gallopamile through the skin, characterized in that for increasing the transdermal permeation lanoline derivatives, in a mixture with polyethyleneglycol ethers of fatty alcohols with longer chains, are contained in said formulation as a penetration enhancing portion.

7. The use of lanoline derivatives, preferably selected from the group consisting of acetylated lanoline, acetylated lanoline alcohol, alkoxylated lanoline, lanoline acid, polyethoxylated lanoline acid, polyethoxylated lanoline alcohol, esters of lanoline acid with short-chain aliphatic alcohols, such as isopropyl lanolate, and esters of lanoline alcohol with long-chain fatty acids, in a mixture with esters of isopropyl alcohols with long-chain fatty acids, preferably selected from the group consisting of isopropyl isostearate, isopropyl laurate, isopropyl linoleate, isopropyl myristate, isopropyl palmitate and isopropyl stearate, and/or with polyethyleneglycol ethers of fatty alcohols with longer chains, preferably selected from the group consisting of reaction products of oleyl alcohol with 2 to 50 moles ethylene oxide, lauryl alcohol with 2 to 40 moles ethylene oxide, cetyl alcohol with 2 to 45 moles ethylene oxide and stearyl alcohol with 2 to 100 moles ethylene oxide as penetration enhancing substances for formulations containing verapamile or gallopamile.

8. Transdermal therapeutic system for the administration of gallopamile or verapamile, containing a formulation according to any one of Claims 2 to 6.

## Claims (Claims for the following Contracting State(s): ES, GR)

1. A process for the use of lanoline derivatives together with polyethyleneglycol ethers of fatty alcohols with longer chains as penetration enhancing substances in formulations containing pharmaceutical substances or other, biologically active substances.

2. A process for producing a formulation to increase the transdermal permeation of pharmaceutical substances or other biologically active substances, characterised by a content of a penetration enhancing portion of lanoline derivatives together with polyethyleneglycol ethers of fatty alcohols with longer chains as penetration enhancing substances.

3. The process for producing a formulation according to Claim 2, characterised in that the lanoline derivatives are selected from the group consisting of acetylated lanoline, acetylated lanoline alcohol, alkoxylated lanoline, lanoline acid, polyethoxylated lanoline acid, polyethoxylated lanoline alcohol, esters of lanoline acid with short-chain aliphatic alcohols such as isopropyl lanolate and esters of lanoline alcohol with long-chain fatty acids.

4. The process for producing a formulation according to Claim 2, characterised in that the polyethyleneglycol ethers of fatty alcohols with longer chains are selected from the group consisting of reaction products of oleyl alcohol with 2 - 50 moles of ethylene oxide, lauryl alcohol with
2 - 40 moles of ethylene oxide, cetyl alcohol with 2 - 45 moles of ethylene oxide and stearyl alcohol with 2 - 100 moles of ethylene oxide.

5. The process for producing a formulation according to any one of Claims 1 to 4, characterized in that the penetration enhancing substance consists of at least 1%-wt., preferably 1 to 60%-wt., of a lanoline derivative, and of up to 99%-wt., preferably 30 to 90%-wt, of a polyethyleneglycol ether of fatty alcohols with longer chains, the sum of the components always amounting to 100.

6. The process for producing a formulation for the administration of verapamile or gallopamile through the skin, characterized in that for increasing the transdermal permeation lanoline derivatives, in a mixture with polyethyleneglycol ethers of fatty alcohols with longer chains, are contained in said formulation as a penetration enhancing portion.

7. A process for the use of lanoline derivatives preferably selected from the group consisting of acetylated lanoline, acetylated lanoline alcohol, alkoxylated lanoline, lanoline acid, polyethoxylated lanoline acid, polyethoxylated lanoline alcohol, esters of lanoline acid with short-chain aliphatic alcohols, such as isopropyl lanolate, and esters of lanoline alcohol with long-chain fatty acids, in a mixture with esters of isopropyl alcohols with long-chain fatty acids, preferably selected from the group consisting of isopropyl isostearate, isopropyl laurate, isopropyl linoleate, isopropyl myristate, isopropyl palmitate and isopropyl stearate, and/or with polyethyleneglycol ethers of fatty alcohols with longer chains, preferably selected from the group consisting of reaction products of oleyl alcohol with 2 to 50 moles ethylene oxide, lauryl alcohol with 2 to 40 moles ethylene oxide, cetyl alcohol with 2 to 45 moles ethylene oxide and stearyl alcohol with 2 to 100 moles ethylene oxide as penetration enhancing substances for formulations containing verapamile or gallopamile.

8. A process for the production of a transdermal therapeutic system for the administration of gallopamile or verapamile, containing a formulation according to any one of Claims 2 to 6.

## Revendications (Revendications pour l'(les) Etat(s) contractant(s) suivant(s): AT, BE, CH, DE, DK, FR, GB, IT, LI, LU, NL, SE)

1. Utilisation de dérives de la lanoline conjointement avec des polyéthylèneglycoléthers d'alcools gras à longues chaînes à titre de substances promotrices de pénétration dans des substances médicamenteuses ou d'autres compositions contenant des substances biologiquement actives.

2. Composition pour l'élévation de la perméation transdermique de substances médicamenteuses ou d'autres substances biologiquement actives, caractérisée par une teneur en fraction promotrice de pénétration de dérivés de la lanoline conjointement avec des polyéthylèneglycoléthers d'alcools gras à longues chaînes à titre de substance promotrice de pénétration.

3. Composition suivant la revendication 2, caractérisée en ce que l'on choisit les dérivés de la lanoline dans le groupe constitué de la lanoline acétylée, de l'alcool de lanoline acétylé, de la lanoline alcoxylée, de l'acide lanolinique, de l'acide lanolinique polyéthoxylé, de l'alcool lanolinique polyéthoxylé, des esters de l'acide lanolinique avec des alcools aliphatiques à courtes chaînes, comme le lanolate d'isopropyle et des esters de l'alcool lanolinique avec des acides gras à longues chaînes.

4. Composition suivant la revendication 2, caractérisée en ce que les polyéthylèneglycoléthers d'alcools gras à longues chaînes sont choisis dans le groupe constitué de produits de la réaction de l'alcool oléylique avec 2 à 50 moles d'oxyde d'éthylène, de l'alcool laurylique avec 2 à 40 moles d'oxyde d'éthylène, de l'alcool cétylique avec 2 à 45 moles d'oxyde d'éthylène et de l'alcool stéarylique avec 2 à 100 moles d'oxyde d'éthylène.

5. Composition suivant l'une quelconque des revendications 1 à 4, caractérisée en ce que la substance promotrice de pénétration se compose d'au moins 1% en poids, de préférence, 1 à 60% en poids, d'un dérivé de la lanoline et de jusqu'à 99% en poids, de préférence, 30 à 90% en poids, d'un polyéthylèneglycoléther d'alcools gras à longues chaînes, où la somme des composants est toujours égale à 100.

6. Composition d'administration du vérapamil ou du gallopamil à travers la peau, caractérisée en ce que, pour augmenter la perméation transdermique, elle contient, à titre de fraction promotrice de pénétration, des dérivés de la lanoline en mélange à des polyéthylèneglycoléthers d'alcools gras à longues chaînes.

7. Utilisation de dérivés de la lanoline, de préférence choisis parmi le groupe constitué de la lanoline acétylée, de l'alcool de lanoline acétylé, de la lanoline alcoxylée, de l'acide lanolinique, de l'acide lanolinique polyéthoxylé, de l'alcool lanolinique polyéthoxylé, des esters de l'acide lanolinique avec des alcools aliphatiques à courtes chaînes, comme le lanolate d'isopropyle et des esters de l'alcool lanolinique avec des acides gras à longues chaînes, en mélange à des esters de l'alcool isopropylique avec des acides gras à longues chaînes, de préférence choisis dans le groupe constitué de l'isostéarate d'isopropyle, du laurate d'isopropyle, du linoléate d'isopropyle, du myristate d'isopropyle, du palmitate d'isopropyle et de stéarate d'isopropyle et/ou des polyéthylèneglycoléthers d'alcools gras à longues chaînes, de préférence choisis dans le groupe constitué de produits de la réaction de l'alcool oléylique avec 2 à 50 moles d'oxyde d'éthylène, de l'alcool laurylique avec 2 à 40 moles d'oxyde d'éthylène, de l'alcool cétylique avec 2 à 45 moles d'oxyde d'éthylène et de l'alcool stéarylique avec 2 à 100 moles d'oxyde d'éthylène, à titre de substances promotrices de pénétration, pour des compositions contenant du vérapamil ou du gallopamil.

8. Système thérapeutique transdermique pour l'administration du gallopamil ou du vérapamil, qui contient une composition suivant l'une quelconque des revendications 2 à 6.

## Revendications (Revendications pour l'(les) Etat(s) contractant(s) suivant(s): ES, GR)

1. Procéd d'utilisation de dérivés de la lanoline conjointement avec des polyéthylèneglycoléthers d'alcools gras à longues chaînes à titre de substances promotrices de pénétration dans des substances médicamenteuses ou d'autres compositions contenant des substances biologiquement actives.

2. Procédé de préparation d'une composition pour l'élévation de la perméation transdermique de substances médicamenteuses ou d'autres substances biologiquement actives, caractérisé par une teneur en fraction promotrice de pénétration de dérivés de la lanoline conjointement avec des polyéthylèneglycoléthers d'alcools gras à longues chaînes à titre de substance promotrice de pénétration.

3. Procédé de préparation d'une composition suivant la revendication 2, caractérisé en ce que l'on choisit les dérivés de la lanoline dans le groupe constitué de la lanoline acétylée, de l'alcool de lanoline acétylé, de la lanoline alcoxylée, de l'acide lanolinique, de l'acide lanolinique polyéthoxylé, de l'alcool lanolinique polyéthoxylé, des esters de l'acide lanolinique avec des alcools aliphatiques à courtes chaînes, comme le lanolate d'isopropyle et des esters de l'alcool lanolinique avec des acides gras à longues chaînes.

4. Procédé de préparation d'une composition suivant la revendication 2, caractérisé en ce que les polyéthylèneglycoléthers d'alcools gras à longues chaînes sont choisis dans le groupe constitué de produits de la réaction de l'alcool oléylique avec 2 à 50 moles d'oxyde d'éthylène, de l'alcool laurylique avec 2 à 40 moles d'oxyde d'éthylène, de l'alcool cétylique avec 2 à 45 moles d'oxyde d'éthylène et de l'alcool stéarylique avec 2 à 100 moles d'oxyde d'éthylène.

5. Procédé de préparation d'une composition suivant l'une quelconque des revendications 1 à 4, caractérisé en ce que la substance promotrice de pénétration se compose d'au moins 1% en poids, de préférence, 1 à 60% en poids, d'un dérivé de la lanoline et de jusqu'à 99% en poids, de préférence, 30 à 90% en poids, d'un polyéthylèneglycoléther d'alcools gras à longues chaînes, où la somme des composants est toujours égale à 100.

6. Procédé de préparation d'une composition d'administration du vérapamil ou du gallopamil à travers la peau, caractérisée en ce que, pour augmenter la perméation transdermique, elle contient, à titre de fraction promotrice de pénétration, des dérivés de la lanoline en mélange à des polyéthylèneglycoléthers d'alcools gras à longues chaînes.

7. Procédé pour l'utilisation de dérivés de la lanoline, de préférence choisis parmi le groupe constitué de la lanoline acétylée, de l'alcool de lanoline acétylé, de la lanoline alcoxylée, de l'acide lanolinique, de l'acide lanolinique polyéthoxylé, de l'alcool lanolinique polyéthoxylé, des esters de l'acide lanolinique avec des alcools aliphatiques à courtes chaînes, comme le lanolate d'isopropyle et des esters de l'alcool lanolinique avec des acides gras à longues chaînes, en mélange à des esters de l'alcool isopropylique avec des acides gras à longues chaînes, de préférence choisis dans le groupe constitué de l'isostéarate d'isopropyle, du laurate d'isopropyle, du linoléate d'isopropyle, du myristate d'isopropyle, du palmitate d'isopropyle et de stéarate d'isopropyle et/ou des polyéthylèneglycoléthers d'alcools gras à longues chaînes, de préférence choisis dans le groupe constitué de produits de la réaction de l'alcool oléylique avec 2 à 50 moles d'oxyde d'éthylène, de l'alcool laurylique avec 2 à 40 moles d'oxyde d'éthylène, de l'alcool cétylique avec 2 à 45 moles d'oxyde d'éthylène et de l'alcool stéarylique avec 2 à 100 moles d'oxyde d'éthylène, à titre de substances promotrices de pénétration, pour des compositions contenant du vérapamil ou du gallopamil.

8. Procédé de préparation d'un système thérapeutique transdermique pour l'administration du gallopamil ou du vérapamil, qui contient une composition suivant l'une quelconque des revendications 2 à 6.
